# EUROPÄISCHE PATENTSCHRIFT

(11) **EP 2 753 291 B1**
(45) Veröffentlichungstag und Bekanntmachung des Hinweises auf die Patenterteilung: **19.06.2019**
(21) Anmeldenummer: 12762248.8
(22) Anmeldetag: 10.09.2012
(51) Int. Cl.: A61K 6/00, A61K 6/083

(54) **DENTALMATERIALIEN AUF BASIS VON VERBINDUNGEN MIT DEBONDING-ON-DEMAND EIGENSCHAFTEN**
DENTAL MATERIALS BASED ON COMPOUNDS HAVING DEBONDING-ON-DEMAND PROPERTIES
MATÉRIAUX DENTAIRES À BASE DE COMPOSÉS PRÉSENTANT DES PROPRIÉTÉS DE DÉCOHÉSION SUR DEMANDE

(30) Priorität: 08.09.2011 EP 11180645
(43) Veröffentlichungstag der Anmeldung: 16.07.2014
(73) Patentinhaber: Ivoclar Vivadent AG, 9494 Schaan (LI)
(72) Erfinder: MOSZNER, Norbert, 9495 Triesen (LI); HIRT, Thomas, CH-9445 Rebstein (CH); RIST, Kai, 6800 Feldkirch (AT); SALZ, Ulrich, 88131 Lindau (DE); WEDER, Christoph, CH-3186 Düdingen (CH); FIORE, Gina, CH-1723 Marly (CH); HEINZMANN, Christian, CH-1700 Fribourg (CH); RHEINBERGER, Volker, FL-9490 Vaduz (LI)
(74) Vertreter: Uexküll & Stolberg
(86) Internationale Anmeldenummer: PCT/EP2012/067680
(87) Internationale Veröffentlichungsnummer: WO 2013/034778

(56) Entgegenhaltungen:
- US-A1- 2006 165 753
- US-A1- 2007 142 494
- US-B2- 7 935 131

## Beschreibung

Die vorliegende Erfindung betrifft die Verwendung von Verbindungen mit Debonding-on-Demand (DoD) Eigenschaften als Komponenten in und zur Herstellung von Adhäsiven und Zementen.

Wiederlösbare Klebeverbindungen haben in verschiedenen Bereichen der Technik eine zunehmende Bedeutung. Beispiele sind das Entfügen von Bauteilen im Rahmen von automatisierten Fertigungsprozessen, die Reparatur von komplexen Bauteilen mit geklebten Teilkomponenten oder die Vereinfachung der stofflichen Trennung beim Recycling solcher Bauteile am Produktlebensende. Das Lösen (debonding) von Klebeverbindungen kann gezielt (on demand) dadurch erreicht werden, dass die Festigkeit der adhäsiven Verbundschicht z.B. durch Erwärmung deutlich verringert wird.

So beschreibt DE 198 32 629 A1 ein Klebstoffsystem zur Bildung reversibler Klebeverbindungen auf Basis von Polyurethanen, Polyharnstoffen oder Epoxidharzen, bei der eine Zusatzkomponente durch Energieeintrag so aktiviert werden kann, dass ein Abbau der Klebstoffkomponenten erfolgt. Beispielsweise können durch Eintrag von Wärme- oder Strahlungsenergie aus blockierten Precursoren organische Basen oder Säuren freigesetzt werden, die einen Abbau des Kleberharzes bewirken.

WO 2010/128042 A1 beschreibt technische Klebstoffzusammensetzungen für lösbare Klebeverbindungen für den Flugzeug- oder Fahrzeugbau, die aus einer üblichen Klebstoffmatrix und einem partikelförmigen Expansionsstoff wie z.B. Azodicarbonamid bestehen. Dabei erfolgt das Lösen der Bauteile durch Erwärmen der Klebeverbindung mindestens auf die Expansionstemperatur des Expansionsstoffes.

WO 02/46260 A1 und WO 2010/002262 A1 beschreiben supramolekulare Polymere, die als Klebstoffe eingesetzt werden können. Diese Kunststoffe zersetzen sich beim Erwärmen in flüssige, niedermolekulare Verbindungen mit niedriger Viskosität. Dieser Prozess ist reversibel, so dass beim Erkalten der Klebstoff wiederhergestellt wird. So können Klebeverbindungen durch Erwärmen bzw. Kühlen reversibel hergestellt und gelöst werden.

Im Dentalbereich ist das Lösen von Klebeverbindungen unter anderem in der Kieferorthopädie von Bedeutung, wo Brackets, die zur Korrektur von Zahnfehlstellungen auf die Zahnoberfläche geklebt werden, nach erfolgter Korrektur ohne Schädigung des Zahnschmelz wieder entfernen werden müssen. Ausserdem wären im Falle der Reparatur oder des vollkommenen Ersatzes von hochfesten keramischen Restaurationen oder Kronen, die mechanisch nur aufwändig entfernbar sind, einfach erweichbare bzw. trennbare Zementverbunde von Vorteil.

Im Zusammenhang mit orthodontischen Anwendungen beschreibt US 2007/0142498 A1 dentale Zusammensetzungen, die thermisch steuerbare Additive wie z.B. thermoplastische Polymere enthalten.

US 2007/0142497 A1 beschreibt dentale Zusammensetzungen auf Basis von Dimethacrylaten mit säurelabilen tertiären Carbonatgruppen und Photosäuren wie z.B. Triarylsulfoniumsalzen. Diese Zusammensetzungen lassen sich mit geeigneten Initiatoren wie etwa dem Bisacylphosphinoxid Irgacure 819 mit Licht im sichtbaren Bereich photochemisch härten (Photobonding) und durch Bestrahlung mit UV-Licht bei erhöhter Temperatur wieder erweichen (photothermisches Debonding).

US 2007/0142494 A1 beschreibt dentale Zusammensetzungen, die Komponenten mit thermolabilen Gruppen mit kovalenter Bindungsstruktur wie Cycloadditionsaddukten oder Oximeestern enthalten, als dentale Adhäsive oder Zemente.

Der Erfindung liegt die Aufgabe zugrunde, adhäsive Dentalwerkstoffe bereitzustellen, die gute Substrathaftung insbesondere auf Zahnhartsubstanz und/oder Dentalkeramiken zeigen und durch Wärmezufuhr ein Debonding vom Substrat gestatten und sich damit vor allem zur Herstellung von Adhäsiven oder Kompositzementen mit Debonding-on-Demand-Eigenschaften eignen.

Diese Aufgabe wird erfindungsgemäss gelöst durch die Verwendung eines Dentalwerkstoffs, der eine thermolabile Verbindung der Formel I enthält:

[(Z¹)ₘ-Q¹-X)]ₖ-T-[Y-Q²-(Z²)ₙ]ₗ Formel I,

in der
- T: für eine thermolabile Einheit steht, die mindestens eine thermolabile Gruppe auf Basis von nicht-kovalenten Wechselwirkungen mit der Formel 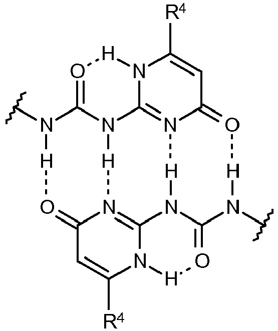 enthält,
- Z¹ und Z²: jeweils unabhängig für eine polymerisierbare Gruppe ausgewählt aus Vinylgruppen, CH₂=CR¹-CO-O- und CH₂=CR¹-CO-NR²- stehen,
- Q¹: jeweils unabhängig entfällt oder für einen (m+1)-wertigen linearen oder verzweigten aliphatischen C₁-C₂₀-Rest steht, der durch -O-, -S-, -CO-O-, -O-CO-, -CO-NR³-, -NR³-CO-, -O-CO-NR³-, -NR³-CO-O- oder -NR³-CO-NR³- unterbrochen sein kann,
- Q²: jeweils unabhängig entfällt oder für einen (n+1)-wertigen linearen oder verzweigten aliphatischen C₁-C₂₀-Rest steht, der durch -O-, -S-, -CO-O-, -O-CO-, -CO-NR³-, -NR³-CO-, -O-CO-NR³-, -NR³-CO-O- oder -NR³-CO-NR³- unterbrochen sein kann,
- X und Y: jeweils unabhängig entfallen oder für -O-, -S-, -CO-O-, -O-CO-, -CO-NR³-, -NR³-CO-, -O-CO-NR³-, -NR³-CO-O- oder -NR³-CO-NR³- stehen,
- R, R¹, R² und R³: jeweils unabhängig für H oder einen C₁-C₇-Alkyl-Rest stehen,
- R⁴: H oder ein C₁-C₁₀-Alkyl-Rest ist,
- k und 1: jeweils unabhängig 0, 1, 2 oder 3 sind und
- m und n: jeweils unabhängig 1, 2 oder 3 sind,
als Adhäsiv oder Zement.

In einer Ausführungsform ist es bevorzugt, dass k und 1 jeweils unabhängig 1, 2 oder 3 sind. In einer anderen Ausführungsform können eines oder beide von Z¹ und Z² entfallen.

Der Hinweis, dass ein Rest durch eine Gruppe wie beispielsweise -O- unterbrochen sein kann, ist so zu verstehen, dass die Gruppe in die Kohlenstoffkette des Restes eingeschoben wird, d.h. beidseitig von Kohlenstoffatomen begrenzt wird. Die Anzahl dieser Gruppen ist daher um mindestens 1 kleiner als die Zahl der Kohlenstoffatome, und die Gruppen können nicht endständig sein. Erfindungsgemäss sind Reste, die nicht durch die genannten Gruppen unterbrochen sind, bevorzugt.

Erfindungsgemäss werden nur solche Verbindungen in Erwägung gezogen, die in mit der chemischen Valenzlehre vereinbar sind.

Besonders bevorzugt sind solche Verbindungen der Formel I, bei denen jeweils unabhängig voneinander
- Q¹: jeweils unabhängig entfällt oder für einen (m+1)-wertigen linearen oder verzweigten aliphatischen C₁-C₁₀-Rest steht, der durch -O-, -CO-O-, -O-CO-, -CO-NR³- oder -NR³-CO- unterbrochen sein kann,
- Q²: jeweils unabhängig entfällt oder für einen (n+1)-wertigen linearen oder verzweigten aliphatischen C₁-C₁₀-Rest steht, der durch -O-, -CO-O-, -O-CO-, -CO-NR³- oder -NR³-CO- unterbrochen sein kann,
- X und Y: jeweils unabhängig entfallen oder für -O-, -CO-O-, -O-CO-, -CO-NR³- oder -NR³-CO- stehen,
- R: jeweils unabhängig H, CH₃ oder C₂H₅ ist,
- R¹: jeweils unabhängig H oder CH₃ ist,
- R²: jeweils unabhängig H, CH₃ oder C₂H₅ ist,
- R³: jeweils unabhängig H, CH₃ oder C₂H₅ ist und/oder
- k, l, m und n: jeweils unabhängig 1 oder 2 sind.

Besonders bevorzugt sind Verbindungen, in denen alle Variablen jeweils eine der oben definierten bevorzugten Bedeutungen haben. In einer Ausführungsform ist die thermolabile Einheit T eine thermolabile Gruppe. Vorzugsweise hat die thermolabile Gruppe die Formel -T¹··T²-, in der T¹ und T² für komplementäre Gruppen stehen, die durch thermolabile nicht-kovalente Wechselwirkungen miteinander verbunden sind.

In einer anderen Ausführungsform ist die thermolabile Einheit T eine oligomere oder polymere Einheit, die mindestens eine thermolabile Gruppe und mindestens eine oligomere, homopolymere oder heteropolymere Kette P enthält. Beispiele für geeignete Ketten P sind Vinyl-, Dien-, Polykondensations- und Polyadditionsoligomere und -polymere, Copolymere und Mischungen davon. Vorzugsweise weisen die Ketten P eine zahlenmittlere Molmasse von 200 bis 20000 g/mol und bevorzugt von 1000 bis 10000 g/mol auf. Besonders bevorzugt sind amorphe oligomere, homopolymere und heteropolymere Ketten mit einer Glasübergangstemperatur von 0°C oder weniger, wie zum Beispiel Ethylen/Butylen-Copolymere, Polyethylenoxid oder Polytetrahydrofuran.

Insbesondere sind solche Verbindungen der Formel I bevorzugt, bei denen die thermolabile Einheit T die Formel III oder IV hat:

[-P-(X¹-Q-Y¹-T¹··T²-Y²-Q-X²-)_{q}]ₚ Formel III,

[-P-(X¹-Q-Y¹-T¹-Y²-Q-X²-)_{q}]ₚ [(-X²-Q-Y²-T²-Y¹-Q-X¹)_{q}-P-]ₚ Formel IV,

in denen
- P: jeweils unabhängig für eine oligomere, homopolymere oder heteropolymere Kette steht, die vorzugsweise eine zahlenmittlere Molmasse von 200 bis 20000 g/mol aufweist,
- T¹ und T²: für Gruppen stehen, die identisch oder verschieden sein können und durch thermolabile nicht-kovalente Wechselwirkungen miteinander verbunden sind,
- Q: jeweils unabhängig entfällt oder für einen zweiwertigen linearen, verzweigten und/oder cyclischen C₁-C₂₀-Rest steht, der ganz oder teilweise ungesättigt und/oder aromatisch sein kann und durch -O-, -S-, -CO-O-, -O-CO-, -CO-NR³-, -NR³-CO-, -O-CO-NR³-, -NR³-CO-O- oder -NR³-CO-NR³- unterbrochen sein kann,
- X¹ und X²: jeweils unabhängig entfallen oder für -O-, -S-, -CO-O-, -O-CO-, -CO-NR³-, -NR³-CO-, -O-CO-NR³-, -NR³-CO-O- oder -NR³-CO-NR³- stehen,
- Y¹ und Y²: jeweils unabhängig entfallen oder für -O-, -S-, -CO-O-, -O-CO-, -CO-NR³-, -NR³-CO-, -O-CO-NR³-, -NR³-CO-O- oder -NR³-CO-NR³- stehen und
- p: unabhängig 1 bis 100 ist und
- q: unabhängig 1, 2, 3 oder 4 ist.

Besonders bevorzugt sind solche Einheiten T, in denen in den Formeln III und IV jeweils unabhängig voneinander
- P: jeweils unabhängig für eine oligomere, homopolymere oder heteropolymere Kette steht, die vorzugsweise eine zahlenmittlere Molmasse von 1000 bis 10000 g/mol aufweist,
- Q: jeweils unabhängig entfällt oder für einen zweiwertigen linearen oder verzweigten C₁-C₁₀-Rest steht, der durch -O-,-S-, -CO-O-, -O-CO-, -CO-NR³-, -NR³-CO-, -O-CO-NR³-, -NR³-CO-O- oder -NR³-CO-NR³- unterbrochen sein kann,
- X¹ und X²: jeweils unabhängig entfallen oder für -O-, -S-, -CO-O-, -O-CO-, -CO-NR³-, -NR³-CO-, -O-CO-NR³-, -NR³-CO-O- oder -NR³-CO-NR³- stehen,
- Y¹ und Y²: jeweils unabhängig entfallen oder für -O-, -S-, -CO-O-, -O-CO-, -CO-NR³-, -NR³-CO-, -O-CO-NR³-, -NR³-CO-O- oder -NR³-CO-NR³- stehen,
- p: unabhängig 1 bis 50, insbesondere 5 bis 50 und besonders bevorzugt 10 bis 50 ist und
- q: unabhängig 1 oder 2 und insbesondere 1 ist.

Besonders bevorzugt sind Verbindungen, in denen alle Variablen jeweils eine der oben definierten bevorzugten Bedeutungen haben.

Erfindungsgemäss bevorzugt sind Verbindungen der Formel I, in denen die thermolabile Einheit T zur Ausbildung supramolekularer Motive befähigt ist. Solche supramolekularen Motive können durch Assoziation von zwei oder mehr chemischen Spezies mittels nichtkovalenter intermolekularer Wechselwirkungen gebildet werden (Supramolecular Chemistry, J.-M. Lehn, Wiley-VCH, 1995 und Supramolecular Chemistry: From Molecules to Nanomaterials, P. A. Gale und J.W. Steed (Hrsg.), Wiley, 2012). Solche Assoziationsprozesse sind die Basis für die Bildung supramolekularer Polymere, bei denen einzelne Polymerketten durch die oben beschriebenen nicht-kovalenten Bindungen zusammengehalten werden (Tom F. A. de Greef et al., Nature 2008, 453, 171-173; T. Aida et al., Science 2012, 335, 813-817). Nicht-kovalente Bindungen sind in der Regel schwächer und dynamischer als kovalente Bindungen. Ihre Bildung ist daher oftmals reversibel, so dass sie beliebig oft, beispielsweise durch Erhöhen der Temperatur, Erniedrigung der Konzentration, oder Erhöhung der Polarität eines Lösungsmittels, gebrochen und wieder gebildet werden können. Solche supramolekularen Motive sind als thermolabile Einheiten besonders geeignet.

Es wurde überraschend gefunden, dass die erfindungsgemäss verwendeten Dentalwerkstoffe, die mindestens eine thermolabile Verbindung der Formel I enthalten, einerseits hervorragende mechanische Eigenschaften sowie eine hervorragende Haftung auf Zahnhartsubstanz und Dentalkeramiken zeigen und sich andererseits durch direkte oder indirekte Wärmezufuhr leicht vom Substrat lösen lassen.

Thermolabile polymerisierbare Verbindungen, die thermolabile Gruppen enthalten, können durch die Reaktion geeignet funktionalisierter Gruppen T¹ bzw. T² mit komplementär funktionalisierten Monomeren dargestellt werden.

So kann zum Beispiel das 2-Ureido-4[1H]-pyrimidon (UPy)-Derivat UPy-CH₂CH₂OC(O)C(CH₃)CH₂ durch Reaktion von 2-Isocyanatoethylmethacrylat und 6-Methylisocytosin (2-Amino-4-hydroxy-6-methyl-pyrimidon) erhalten werden:

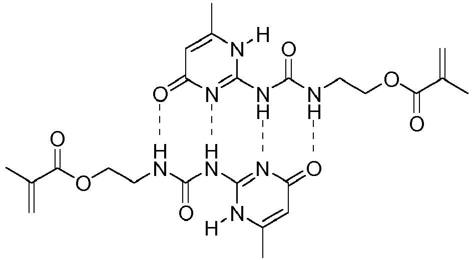

Die erfindungsgemäss verwendeten Verbindungen der Formel I mit einer oligomeren oder polymeren thermolabilen Einheit T lassen sich analog zu an sich bekannten Syntheseverfahren herstellen.

So können α,ω-OH-terminierte Oligomere oder Polymere, wie Polysiloxane, Polyether, Polyester oder Polycarbonate, mit einem Überschuss an einer Verbindung umgesetzt werden, in der eine Gruppe T¹ bzw. T² kovalent mit einer funktionalen Gruppe verbunden ist, welche mit Hydroxylgruppen reagieren kann, beispielsweise einer Isocyanatgruppe.

Beispiel: Umsetzung eines α,ω-OH-terminierten Polymers mit einem Überschuss an 2-(6-Isocyanatohexylaminocarbonylamino)-6-methyl-4[1H]pyrimidinon zu einem jeweils endständig mit 2-Ureido-4[1H]-pyrimidinon-Gruppen funktionalisierten Polymer, das zur Ausbildung von Wasserstoffbrückenbindungen geeignet ist (B. J. B. Folmer et al., Adv. Mater. 2000, 12, 874).

Umgekehrt können α,ω-OH-terminierte Oligomere oder Polymere zunächst mit einem Überschuss einer Verbindung umgesetzt werden, in der eine Linkergruppe an beiden Enden mit einer funktionalen Gruppe verbunden ist, welche mit Hydroxylgruppen reagieren kann, beispielsweise einer Isocyanatgruppe. Anschliessend kann das so erhaltene α,ω-OH-funktionalisierte Oligomer bzw. Polymer mit einer Verbindung umgesetzt werden, in der eine Gruppe T¹ bzw. T² kovalent mit einer funktionalen Gruppe wie einer Hydroxylgruppe verbunden ist.

Alternativ können α,ω-OH-terminierte Oligomere oder Polymere etwa mittels Mitsunobu-Reaktion mit einer Verbindung umgesetzt werden, in der eine Gruppe T¹ bzw. T² kovalent mit einer funktionalen Gruppe wie einer Hydroxylgruppe verbunden ist.

In analoger Weise kann weiteres α,ω-OH-terminiertes Oligomer bzw. Polymer zunächst an einem Ende mit einer Gruppe T¹ bzw. T² funktionalisiert und dann am anderen Ende mit einer polymerisierbaren Gruppe funktionalisiert werden oder umgekehrt. Das so erhaltene gemischt funktionalisierte Oligomer bzw. Polymer kann dann zusammen mit an beiden Enden mit Gruppen T¹ bzw. T² funktionalisiertem Oligomer bzw. Polymer in an sich bekannter Weise zu einer Verbindung der Formel I umgesetzt werden.

Alternativ können zur Herstellung erfindungsgemäss verwendeter Verbindungen der Formel I mit einer oligomeren oder polymeren thermolabilen Einheit T thermolabile polymerisierbare Verbindungen, die thermolabile Gruppen enthalten, wie oben beschrieben hergestellt und durch Copolymerisation etwa mit monofunktionellem Methacrylatmonomeren umgesetzt werden.

Die erfindungsgemäss verwendeten Dentalmaterialien enthalten neben der thermolabilen Verbindung der Formel I vorzugsweise ein oder mehrere (zusätzliche) radikalisch polymerisierbare Monomere (Co-Monomere), insbesondere mono- oder polyfunktionelle (Meth)acrylsäurederivate. Unter monofunktionellen (Meth)acrylsäurederivaten werden Verbindungen mit einer, unter polyfunktionellen (Meth)acrylsäurederivaten Verbindungen mit zwei oder mehr, vorzugsweise 2 bis 4 (Meth)acrylsäuregruppen verstanden. Polyfunktionelle Monomere haben eine vernetzende Wirkung.

Erfindungsgemäss bevorzugte mono- oder polyfunktionelle (Meth)acrylsäurederivate sind Methyl-, Ethyl-, Hydroxyethyl-, Butyl-, Benzyl-, Tetrahydrofurfuryl- oder Isobornyl(meth)acrylat, Bisphenol-A-di(meth)acrylat, Bis-GMA (ein Additionsprodukt aus Methacrylsäure und Bisphenol-A-diglycidylether), UDMA (ein Additionsprodukt aus 2-Hydroxyethylmethacrylat (HEMA) und 2,2,4-Trimethylhexamethylendiisocyanat), Di-, Tri- oder Tetraethylenglycoldi(meth)acrylat, Trimethylolpropantri(meth)acrylat, Pentaerythrittetra(meth)acrylat, Glycerindi(meth)acrylat, 1,4-Butandioldi(meth)acrylat, 1,10-Decandioldi(meth)acrylat und 1,12-Dodecandioldi(meth)acrylat.

Besonders bevorzugte mono- oder polyfunktionelle (Meth)acrylsäurederivate sind N-mono- oder -disubstitiuierte Acrylamide wie N-Ethylacrylamid, N,N-Dimethacrylamid, N-(2-Hydroxyethyl)acrylamid oder N-Methyl-N-(2-hydroxyethyl)acrylamid, N-monosubstituierte Methacrylamide wie N-Ethylmethacrylamid oder N-(2-Hydroxyethyl)-methacrylamid sowie N-Vinylpyrrolidon und Allylether. Diese Monomere zeichnen sich durch eine hohe Hydrolysestabilität aus und eignen sich aufgrund ihrer relativ geringen Viskosität besonders als Verdünnermonomere.

Bevorzugte polyfunktionelle (Meth)acrylsäurederivate mit hoher Hydrolysestabilität sind vernetzende Pyrrolidone wie 1,6-Bis(3-vinyl-2-pyrrolidonyl)-hexan, Bisacrylamide wie Methylen- oder Ethylenbisacrylamid und Bis(meth)acrylamide wie N,N'-Diethyl-1,3-bis-(acrylamido)-propan, 1,3-Bis(methacrylamido)-propan, 1,4-Bis-(acrylamido)-butan oder 1,4-Bis(acryloyl)-piperazin, die durch Umsetzung aus den entsprechenden Diaminen mit (Meth)acrylsäurechlorid synthetisiert werden können.

Erfindungsgemäss als Co-Monomere besonders geeignet sind auch thermolabile Vernetzermonomere. Darunter werden Vernetzermonomere verstanden, die mindestens eine thermolabile kovalente Gruppe zwischen zwei polymerisierbaren Gruppen aufweisen. Beispiele sind polyfunktionelle (Meth)acrylate oder (Meth)acrylamide mit mindestens einer thermolabilen Gruppe zwischen zwei (Meth)acrylgruppen. Dabei kommen als thermolabile kovalente Gruppen insbesondere thermolabile Cycloadditionsaddukte wie Diels-Alder-Addukte, Hetero-Diels-Alder-Addukte sowie thermolabile Alkoxyamin-, Oximester-, Oximurethan- oder Azogruppen in Frage. Beispiele sind Diels-Alder-Addukte wie das Diels-Alder-Addukt aus Furfurylmethacrylat und N-(3-(Methacryloyloxy)propyl)-maleinimid, die Umsetzungsprodukte von N-Hydroxy-(meth)acrylamid mit Di- oder Triisocyanaten wie Hexamethylen-1,6-diisocyanat (HDI), 2,2,4-Trimethylhexamethylen-1,6-diisocyanat oder dem HDI-Trimer sowie Produkte, die durch stöchiometrische Umsetzung von Di- oder Triisocyanaten mit 1-Hydroxymethylacrylsäureestern wie 1-Hydroxymethylacrylsäureethylester oder mit β-Ketoester(meth)acrylaten wie 2-Acetoacetoxyethylmethacrylat erhalten werden. Besonders geeignet sind auch gasfreisetzende thermolabile Vernetzermonomere. Beispiele sind die Veresterungsprodukte von Azobis(4-cyanovaleriansäure) mit Hydroxyalkyl(meth)acrylaten wie Hydroxyethyl(meth)acrylat oder Hydroxypropyl(meth)acrylat oder mit N-(Hydroxyalkyl)(meth) acrylamiden wie N-(5-Hydroxypentyl)methacrylamid oder N-Methyl-N-(2-hydroxyethyl)acrylamid.

Die erfindungsgemäss verwendeten Dentalwerkstoffe können neben der thermolabilen Verbindung der Formel I und ggf. den oben genannten Co-Monomeren vorzugweise auch radikalisch polymerisierbare, säuregruppenhaltige Monomere (Haftmonomere) enthalten. Bevorzugte Säuregruppen sind Carbonsäuregruppen, Phosphonsäuregruppen, Phosphorsäuregruppen und Sulfonsäuregruppen.

Bevorzugte Monomere mit polymerisierbaren Carbonsäuren sind Maleinsäure, Acrylsäure, Methacrylsäure, 2-(Hydroxymethyl)acrylsäure, 4-(Meth)acryloyloxyethyltrimellitsäureanhydrid, 10-Methacryloyloxydecylmalonsäure, N-(2-Hydroxy-3-methacryloyloxypropyl)-N-phenylglycin und 4-Vinylbenzoesäure.

Bevorzugte Monomere mit polymerisierbaren Phosphonsäuregruppen sind Vinylphosphonsäure, 4-Vinylphenylphosphonsäure, 4-Vinylbenzylphosphonsäure, 2-Methacryloyloxyethylphosphonsäure, 2-Methacrylamidoethylphosphonsäure, 4-Methacrylamido-4-methyl-pentylphosphonsäure, 2-[4-(Dihydroxyphosphoryl)-2-oxa-butyl]-acrylsäure, 2-[4-(Dihydroxyphosphoryl)-2-oxa-butyl]-acrylsäureethyl- und -2,4,6-trimethylphenylester.

Bevorzugte Monomere mit polymerisierbaren Phosphorsäuregruppen sind 2-Methacryloyloxypropylmono- oder -dihydrogenphosphat, 2-Methacryloyloxyethylmono- oder -dihydrogenphosphat, 2-Methacryloyloxyethylphenyl-hydrogenphosphat, Dipentaerythritolpentamethacryloyloxyphosphat, 10-Methacryloyloxydecyl-dihydrogenphosphat, Phosphorsäuremono-(1-acryloyl-piperidin-4-yl)-ester, 6-(Methacrylamido)hexyldihydrogenphosphat und 1,3-Bis-(N-acryloyl-N-propylamino)-propan-2-yl-dihydrogenphosphat.

Bevorzugte Monomere mit polymerisierbaren Sulfonsäuregruppen sind Vinylsulfonsäure, 4-Vinylphenylsulfonsäure und 3-(Methacrylamido)-propylsulfonsäure.

Vorzugsweise werden Mischungen der Verbindung der Formel I mit den vorstehend genannten Monomeren verwendet. Bevorzugte Mischungen enthalten bezogen auf das Gesamtgewicht der Mischung:
1 bis 90 Gew.-%, vorzugsweise 5 bis 80 Gew.-%, besonders bevorzugt 5 bis 70 Verbindung der Formel I,
0 bis 70 Gew.-%, vorzugsweise 1 bis 60 Gew.-%, besonders bevorzugt 5 bis 50 und ganz besonders bevorzugt 10 bis 30 Gew.-% Co-Monomer und insbesondere mono- und/oder polyfunktionelle (Meth)acrylate,
0 bis 70 Gew.-%, vorzugsweise 1 bis 60 Gew.-% und besonders bevorzugt 5 bis 50 Gew.-% thermolabiles Vernetzermonomer und 0 bis 40 Gew.-%, vorzugsweise 1 bis 30 Gew.-% und besonders bevorzugt 5 bis 20 Gew.-% Haftmonomer.

Besonders bevorzugte Mischungen (jeweils bezogen auf das Gesamtgewicht der Mischung) sind in der nachfolgenden Tabelle wiedergegeben:

| Komponente (Gew.-%) | A | B | C | D | E | F |
|---|---|---|---|---|---|---|
| Verbindung der Formel I | 1-90 | 5-80 | 5-70 | 5-70 | 5-70 | 5-70 |
| Co-Monomer, insbesondere mono- und/oder polyfunktionelles (Meth)acrylat | 0-70 | 0-60 | 1-60 | 5-60 | 5-50 | 0-30 |
| thermolabiles Vernetzermonomer | 0-70 | 0-50 | 0-50 | 5-50 | 5-50 | 5-50 |
| Haftmonomer | 0-40 | 0-30 | 0-30 | 0-20 | 0-20 | 0-30 |

Ausserdem enthalten die erfindungsgemäss verwendeten Dentalwerkstoffe vorzugsweise auch einen Initiator für die radikalische Polymerisation.

Zur Initiierung der radikalischen Photopolymerisation werden vorzugsweise Benzophenon, Benzoin sowie deren Derivate oder α-Diketone oder deren Derivate wie 9,10-Phenanthrenchinon, 1-Phenyl-propan-1,2-dion, Diacetyl oder 4,4'-Dichlorbenzil eingesetzt. Besonders bevorzugt werden Campherchinon und 2,2-Dimethoxy-2-phenylacetophenon und ganz besonders bevorzugt α-Diketone in Kombination mit Aminen wie 4-(Dimethylamino)-benzoesäureester, N,N-Dimethylaminoethylmethacrylat, N,N-Dimethyl-sym.-xylidin oder Triethanolamin als Reduktionsmittel verwendet. Besonders geeignet sind auch Norrish-Typ-I-Photoinitiatoren, insbesondere Acyl- oder Bisacylphosphinoxide, Monoacyltrialkyl- oder Diacyldialkylgermanium-Verbindungen wie Benzoyltrimethylgermanium, Dibenzoyldiethylgermanium oder Bis-(4-methoxybenzoyl)diethylgermanium. Dabei lassen sich auch Mischungen der verschiedenen Photoinitiatoren wie beispielsweise Dibenzoyldiethylgermanium in Kombination mit Campherchinon und 4-Dimethylaminobenzoesäureethylester einsetzen.

Als Initiatoren für eine bei Raumtemperatur durchgeführte Polymerisation werden vorzugsweise Redox-Initiatorkombinationen wie beispielsweise Kombinationen von Benzoylperoxid mit N,N-Dimethylsym.-xylidin oder N,N-Dimethyl-p-toluidin verwendet. Darüber hinaus sind auch Redoxsysteme bestehend aus Peroxiden und solchen Reduktionsmitteln wie z.B. Ascorbinsäure, Barbituraten oder Sulfinsäuren besonders geeignet.

Die erfindungsgemässen Dentalwerkstoffe können zudem ein thermisch gasfreisetzendes Additive enthalten. Geeignete gasfreisetzende Additive sind z.B. Azo-Verbindungen wie Azodicarbonamid, 2,2'-Azobisisobutyronitril oder 2,2'-Azobis(4-cyano-pentansäure), N-Nitrosoverbindungen, Hydrazide wie Benzolsulfonylhydrazid, Peroxide wie Dicumolperoxid oder Acetondicarbonsäure. Beispiele für solche Verbindungen sind etwa in St. Quinn, Plastics, Additives & Compounding 2001, 3, 16-21 beschrieben. Dabei kann die Zerfallstemperatur beispielsweise im Falle der Azoverbindungen in an sich bekannter Weise durch das Substituentenmuster eingestellt werden (vgl. D. Braun, R. Jakobi, Monatshefte Chemie 1982, 113, 1403-1414).

Weiterhin können die erfindungsgemäss verwendeten Dentalwerkstoffe ein Additiv enthalten, das eingestrahlte elektromagnetische Strahlung in Wärme umwandeln kann. Solche sogenannten Strahlung-Wärme-Umwandler sind organische, anorganische oder metallorganische Stoffe oder Hybridkomponenten, die in der Lage sind, UV-, NIR- oder IR-Strahlung, sichtbares Licht, Mikrowellen- oder Radiowellenstrahlung in Wärme umzuwandeln, um thermolabile Gruppen zu spalten. Beispiele hierfür sind UV-, NIR oder IR-Strahlung absorbierende Farbstoffe und Pigmente. Beispiele für im IR-Bereich absorbierende Farbstoffe sind Azo-, Methin-, Anthrachinon oder Porphyrinfarbstoffe. Beispiele für NIR-Strahlung absorbierende Pigmente sind Antimon- und Indiumzinnoxid, Phthalocyaninpigmente, Russ, Ni- und Pt-Dithiolen-Komplexe. Beispiele für im UV-Bereich absorbierende Verbindungen sind Benzotriazole, Triazine, Benzophenone, Cyanoacrylate, Salicylsäurederivate und Hindered Amine Light Stabilizers (HALS). Beispiele für Additive, die im Frequenzbereich der Mikrowellen (1 bis 300 GHz) oder Radiowellen (10 kHz bis 1 GHz) absorbieren, sind ferromagnetische keramische Stoffe, sogenannte Ferrite, die aus den Eisenoxiden Hämatit (Fe₂O₃) oder Magnetit (Fe₃O₄) und weiteren Oxiden beisielweise der Metalle Zn, Mn, oder Ni aufgebaut und als Pulver kommerziell verfügbar sind.

Weiterhin enthalten die erfindungsgemässen Dentalwerkstoffe zur Verbesserung der mechanischen Eigenschaften oder zur Einstellung der Viskosität vorzugsweise auch organische oder anorganische Füllstoffpartikel. Bevorzugte anorganische partikuläre Füllstoffe sind amorphe kugelförmige Materialien auf Basis von Oxiden wie ZrO₂ und TiO₂ oder Mischoxiden aus SiO₂, ZrO₂ und/oder TiO₂ mit einer mittleren durchschnittlichen Partikelgrösse von 0,005 bis 2 µm, vorzugsweise 0,1 bis 1 µm, nanopartikuläre oder mikrofeine Füllstoffe wie pyrogene Kieselsäure oder Fällungskieselsäure mit einer mittleren durchschnittlichen Partikelgrösse von 5 bis 200 nm, vorzugsweise 10 bis 100 nm, Minifüllstoffe wie Quarz-, Glaskeramik- oder Glaspulver mit einer durchschnittlichen Teilchengrösse von 0,01 bis 10 µm, vorzugsweise 0,1 bis 1 µm, sowie röntgenopake Füllstoffe wie Ytterbiumtrifluorid oder nanopartikuläres Tantal(V)-oxid bzw. Bariumsulfat mit einer mittleren durchschnittlichen Partikelgrösse von 10 bis 1000 nm, vorzugsweise 100 bis 300 nm.

Ausserdem können die erfindungsgemäss verwendeten Dentalwerkstoffe weitere Additive enthalten, vor allem Lösungsmittel wie Wasser oder Ethanol bzw. entsprechende Lösungsmittelgemische, sowie beispielsweise Stabilisatoren, Aromastoffe, Farbmittel, mikrobiozide Wirkstoffe, fluoridionenabgebende Additive, optische Aufheller oder Weichmacher.

Besonders bevorzugt sind Dentalwerkstoffe auf Basis einer thermolabilen Verbindung der Formel I, welche die folgenden Bestandteile enthalten:
a) 0,1 bis 50 Gew.-%, insbesondere 1 bis 40 Gew.-%, bevorzugt 2 bis 30 Gew.-% und besonders bevorzugt 5 bis 30 Gew.-% Verbindung der Formel I,
b) 0,01 bis 10 Gew.-%, bevorzugt 0,1 bis 3,0 Gew.-% und besonders bevorzugt 0,2 bis 2 Gew.-% Initiator,
c) 0 bis 80 Gew.-%, bevorzugt 1 bis 60 Gew.-% und besonders bevorzugt 5 bis 50 Gew.-% Co-Monomer,
d) 0 bis 30 Gew.-%, bevorzugt 0,5 bis 15 Gew.-% und besonders bevorzugt 1 bis 5 Gew.-% Haftmonomer,
e) 0 bis 80 Gew.-% Füllstoff,
f) 0 bis 70 Gew.-% Lösungsmittel.

Der bevorzugte Füllstoffgehalt richtet sich dabei nach der gewünschten Anwendung. Adhäsive enthalten vorzugsweise 0 bis 20 Gew.-% und Zemente und Komposite vorzugsweise 20 bis 80 Gew.-% Füllstoff.

Dies gilt ebenso für den Lösungsmittelgehalt. Adhäsive enthalten vorzugweise bevorzugt 0 bis 60 Gew.-% und besonders bevorzugt 1 bis 50 Gew.-% Lösungsmittel. Dentalmaterialien, die Wasser als Lösungsmittel enthalten, sind bevorzugt. Besonders bevorzugt sind Dentalmaterialien, die 0 bis 20 Gew.-% und insbesondere 1 bis 10 Gew.-% Wasser enthalten.

Die Debonding-Eigenschaften der erfindungsgemässen Dentalwerkstoffe können durch die Zusammensetzung der Materialien gezielt beeinflusst werden. Die Einstellung einer für einen bestimmten Zweck geeigneten Zusammensetzung gehört zum allgemeinen Wissen und Können des Fachmanns. So nimmt mit der verwendeten Konzentration an thermolabilen Komponenten, d.h. insbesondere der thermolabilen Verbindung der Formel I sowie ggf. der thermolabilen Vernetzermonomere und gasfreisetzenden Additive, die Fähigkeit zum gezielten Debonding durch Erwärmen zu. Weiterhin lassen sich die Debonding-Eigenschaften auch durch die Auswahl der Co-Monomere variieren, wobei mit dem Anteil an vernetzenden Monomeren oder durch Zugabe von monofunktionellen Monomeren die Vernetzungsdichte und damit auch die Festigkeit und der E-Modul variiert werden können.

Die erfindungsgemäss verwendeten Dentalmaterialien auf Basis der thermolabilen Verbindung der Formel I können insbesondere zur reversiblen Befestigung beispielsweise von Brackets, Kronen oder Verblendungen (Veneers) verwendet werden. Dabei erfolgt vorzugsweise zunächst die Bildung eines Verbundes durch Aushärtung von Materialien (Adhäsiv oder Zement) auf Basis der thermolabilen Verbindung der Formel I. Alternativ können die erfindungsgemäss verwendeten oligomeren oder polymeren thermolabilen Verbindungen der Formel I auch direkt eingesetzt werden, beispielsweise in Form eines Pulvers, wobei sie zur Bildung eines Verbundes kurzzeitig auf eine Temperatur erwärmt werden, die über der Temperatur liegt, bei der die Spaltung der thermolabilen Bindungen einsetzt. Dabei kann die Energiezufuhr mittels elektromagnetischer Strahlung erfolgen. Vorzugsweise wird eine oligomere oder polymere thermolabile Verbindung der Formel I dazu mit einem Additiv kombiniert, das die eingestrahlte elektromagnetische Strahlung in Wärme umwandeln kann. Gemäss einer bevorzugten Ausführungsform erfolgt eine gezielte Energiezufuhr über eine IR-Strahlungsquelle oder einen Laser. Gemäss einer anderen bevorzugten Ausführungsform erfolgt die Energiezufuhr mittels UV-Strahlung, insbesondere mit einer Wellenlänge im Bereich von 320 bis 390 nm, vorzugsweise in Gegenwart eines UV-Absorbers. Ausserdem kann beim Einbau von ferromagnetischen Teilchen wie beispielsweise ferromagnetischen Nanopartikeln in die erfindungsgemäss verwendeten Dentalmaterialien eine induktive Erwärmung durch Einwirkung eines magnetischen Wechselfeldes erreicht werden. Zum Debonding müssen die geklebten Teile ebenfalls, insbesondere wie vorstehend beschrieben, kurzzeitig auf eine Temperatur erwärmt werden, die über der Temperatur liegt, bei der die Spaltung der thermolabilen Bindungen einsetzt.

Gegenstand der Erfindung ist auch die Verwendung einer thermolabilen Verbindung der Formel I oder einer wie oben beschriebenen Zusammensetzung zur Herstellung von Adhäsiven oder Zementen, besonders bevorzugt selbstätzenden Adhäsiven oder Zementen.

Im Folgenden wird die Erfindung anhand von Ausführungsbeispielen näher erläutert.

### Ausführungsbeispiele

### Beispiel 1

### Synthese einer oligomeren thermolabilen Wasserstoffbrückenverbindung UPy-Kraton

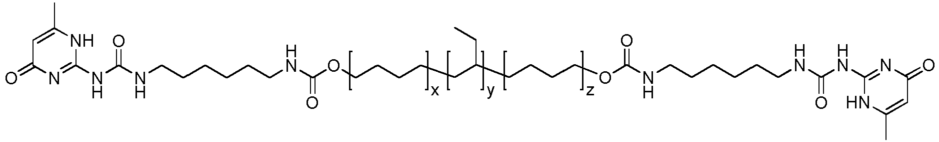

Hydroxy-terminiertes hydriertes Poly(ethylen-co-butylen) (Krasol HLBH-P 3000, Cray Valley, mittlere Molmasse 3500 g/mol, 10,0 g) wurde 2 Tage im Vakuumtrockenschrank bei 65 °C und 400 mbar getrocknet und danach in 100 ml wasserfreiem Chloroform durch Rühren unter Stickstoffatmosphäre gelöst. 2-(6-Isocyanatohexylaminocarbonylamino)-6-methyl-4[1H]pyrimidin (UPy-Isocyanat, 3,32 g, 11,3 mmol) wurde in 30 ml trocknem Chloroform gelöst und der Krasol-Lösung zugegeben, und die Reaktionsmischung wurde auf 40 °C erwärmt. Anschliessend wurden als Katalysator 2 Tropfen Dibutylzinndilaurat zugegeben und die Mischung wurde erhitzt und 16 h unter Rückfluss gerührt. Nach Abkühlen auf 50 °C wurden 3 g 3-Aminopropylsilikat zugegeben und die Reaktionsmischung wurde nochmals erhitzt und 3 h unter Rückfluss gerührt. Danach wurde die Reaktionsmischung auf Raumtemperatur abgekühlt und über Nacht gerührt. Die Mischung wurde bei Raumtemperatur zentrifugiert (7000 rpm, 5 min), die festen Bestandteile wurden abgetrennt und der Rückstand wurde zweimal mit Chloroform gewaschen. Die organischen Phasen wurden vereinigt und das Lösungsmittel wurde am Rotationsverdampfer abgetrennt. Das Rohprodukt wurde dann am Hochvakuum über Nacht bei Raumtemperatur getrocknet. Man erhielt 9,78 g (76% Ausbeute) UPy-Kraton als transparenten, gummiartigen Feststoff.

¹H-NMR (360 MHz, CDCl₃) : δ = 13,14 (s, 1H), 11,88 (s, 1H), 10,12 (s, 1H), 5,86 (s, 1H), 4,84 (s, 1H), 4,06 (s, 3H), 3,20 (m, 8H), 2,23 (s, 6H, CH3), 1,67-0,92 (m, 436H), 0,92-0,50 (m, 132H). ¹³C-NMR (91 MHz, CDCl₃): δ = 183,06, 148,47, 135,73, 107,11, 103,50, 39,27, 38,81, 38,27, 36,50, 33,89, 33,64, 31,08, 30,62, 30,16, 27,18, 26,99, 26,83, 26,44, 26,27, 11,28, 11,05, 10,95, 10,78, 10,67, 10,35. IR (cm⁻¹): 2959, 2921, 2872, 2852, 2270, 1700, 1666, 1589, 1527, 1460, 1379, 1304, 1254, 1139, 1041, 942, 843, 761, 744, 721, 621, 612, 602, 593, 586. GPC: Mₙ = 4000 g/mol, M_{w} = 7600 g/mol, Polydispersität (M_{w}/Mₙ) : 1,90.

### Beispiel 2

### Zementieren von Zahnkronen mit UPy-Kraton aus Beispiel 1

5 Straumann Anatomical IPS e.max Abutments (Ivoclar Vivadent AG) wurden jeweils auf Laboranaloge für die zementierte und herausnehmbare Prothetik montiert, auf eine Stumpfhöhe von 4 mm gekürzt und die Stumpfenden gerundet. Passend hierzu wurden 5 Fräskronen aus e.max ZirCAD (Ivoclar Vivadent AG) unter Einstellen eines Zementspaltes von 100 µm hergestellt. UPy-Kraton aus Beispiel 1 wurde unter Kühlung zu Pulver vermahlen. Die Kronen wurden mit 20 mg gepulvertem UPy-Kraton gefüllt und 5 min auf 80 °C temperiert. Hierbei schmolz das UPy-Kraton auf. Anschliessend wurden die ebenfalls auf 80°C temperierten Abutments unter leichtem Drehen von oben in die Kronen eingeführt. Die zementierten Kronen wurden zur Vervollständigung des Verbundes bei 80 °C für 3 min mit 20 N belastet und anschliessend mindestens 10 min unter Beibehaltung der Last auf Raumtemperatur abgekühlt. Die Messung der Abzugskräfte erfolgte frühestens 1 h nach Probenherstellung.

Für die Ermittlung der Abzugskräfte wurden die Kronen in einer Universalzugprüfmaschine (Zwick-Roell Z010) eingespannt. Die Kronen wurden anschliessend mit einer konstanten Geschwindigkeit von 1.0 mm/min vom Abutment abgezogen und die jeweils auftretende Maximalkraft registriert. Es wurde eine Abzugskraft von 103.0 ± 23.7 N ermittelt.

Durch Erwärmen der zementierten Kronen auf 80 °C konnten diese kraftlos von den Stümpfen entfernt werden.

### Beispiel 3A (Vergleich)

### Optisches Verkleben von transparenten Substraten mit UPy-Kraton aus Beispiel 1

UPy-Kraton aus Beispiel 1 wurde zu einem ca. 90 pm dünnen Film verarbeitet, welcher auf einer Fläche von ca. 10x10 mm zwischen zwei Quarzgläsern platziert wurde. Die zu verklebende Stelle wurde mit UV-Licht aus einer Punktlichtquelle (Dr. Hoenle, Wellenlängenbereich λ = 320-390 nm, Intensität 900 mW cm⁻¹) mit Impulsen von 2x 60 s bestrahlt. Dabei wurde keine Klebeverbindung erreicht.

### Beispiel 3B

### Optisches Verkleben von transparenten Substraten mit einer Mischung von UPy-Kraton aus Beispiel 1 und einem UV Absorber

UPy-Kraton aus Beispiel 1 wurde mit 0,25 Gew.-% des UV-Absorbers 2-(5-Chlor-2H-benzotriazol-2-yl)-6-(1,1-dimethylethyl)-4-methylphenol (Tinuvin 326, Ciba) vermischt. Diese Mischung wurde zu einem ca. 90 pm dünnen Film verarbeitet, welcher auf einer Fläche von ca. 10x10 mm zwischen zwei Quarzgläsern platziert wurde. Die zu verklebende Stelle wurde mit UV-Licht aus einer Punktlichtquelle (Dr. Hoenle, Wellenlängenbereich λ = 320-390 nm; Intensität 900 mW cm⁻¹) mit Impulsen von 2x 60 s bestrahlt. Dabei wurde eine luftblasenfreie Verbindung mit guter Haftung zwischen den beiden Substraten erreicht. Die Messung der Abzugskräfte erfolgte durch Messung in einer Universalzugprüfmaschine (Zwick-Roell Z010). Es wurde eine Abzugskraft von etwa 1 MPa ermittelt.

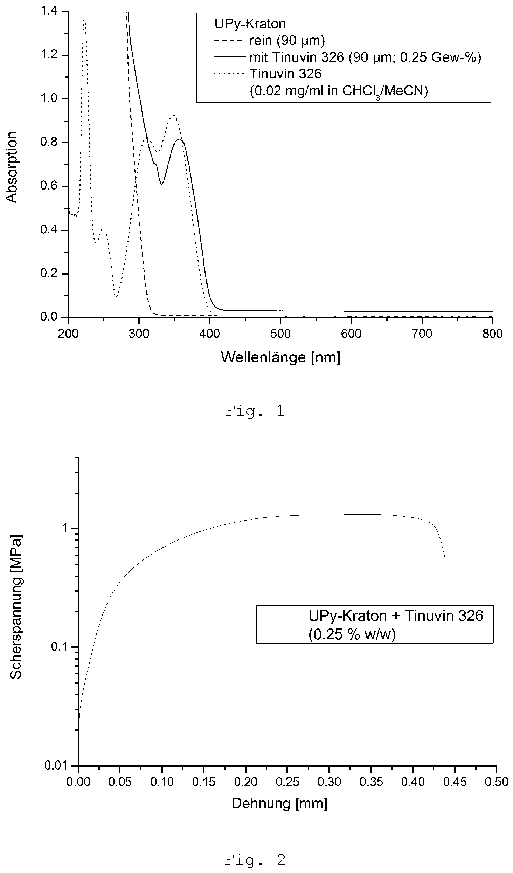

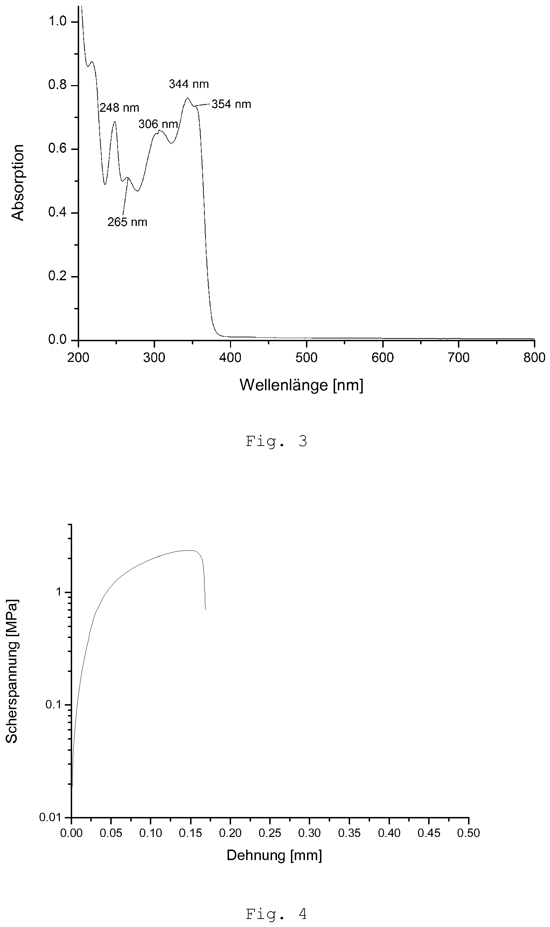

Fig. 1 zeigt UV-Vis-Absorptionsspektren von ca. 90 pm dünnen Filmen aus UPy-Kraton aus Beispiel 1 ("rein") bzw. einer Mischung von UPy-Kraton aus Beispiel 1 mit 0,25 Gew.-% des UV-Absorbers Tinuvin 326 ("mit Tinuvin 326") sowie einer Lösung von 0,02 mg/ml Tinuvin 326 in einer Mischung von Chloroform und Acetonitril ("Tinuvin 326").

Fig. 2 zeigt das Ergebnis der Zugprüfung der Klebeverbindung aus Beispiel 3B.

### Beispiel 3C (Vergleich)

### Optisches Entkleben einer Klebverbindung von transparenten Substraten mit UPy-Kraton aus Beispiel 1

UPy-Kraton aus Beispiel 1 wurde zu einem ca. 90 pm dünnen Film verarbeitet, welcher auf einer Fläche von ca. 10x10 mm zwischen zwei Quarzgläsern platziert wurde. Die zu verklebende Stelle wurde mittels eines Heissluftföns so lange erhitzt, bis eine luftblasenfreie Verbindung entstand. Dabei wurde eine gute Haftung zwischen den beiden Substraten erreicht. Die Messung der Abzugskräfte erfolgte durch Messung in einer Universalzugprüfmaschine (Zwick-Roell Z010). Es wurde eine Abzugskraft von etwa 1,1 MPa ermittelt.

Anschliessend wurde die Probe in eine Universalzugprüfmaschine (Zwick-Roell Z010) eingespannt und gespannt, so dass eine Kraft von etwa 53 N auf die Klebstelle wirkte. Die Klebstelle wurde mit UV-Licht aus einer Punktlichtquelle (Dr. Hoenle, Wellenlängenbereich λ = 320-390 nm; Intensität 900 mW cm⁻¹) 300 s lang bestrahlt. Dabei wurde die Klebeverbindung jedoch nicht aufgelöst.

### Beispiel 3D

### Optisches Entkleben einer Klebeverbindung von transparenten Substraten mit einer Mischung von UPy-Kraton aus Beispiel 1 und einem UV Absorber

UPy-Kraton aus Beispiel 1 wurde mit 0,25 Gew.-% des UV-Absorbers 2-(5-Chlor-2H-benzotriazol-2-yl)-6-(1,1-dimethylethyl)-4-methylphenol (Tinuvin 326, Ciba) vermischt. Diese Mischung wurde zu einem ca. 90 µm dünnen Film verarbeitet, welcher auf einer Fläche von ca. 10x10 mm zwischen zwei Quarzgläsern platziert wurde. Die zu verklebende Stelle wurde mittels eines Heissluftföns so lange erhitzt, bis eine luftblasenfreie Verbindung entstand. Dabei wurde eine gute Haftung zwischen den beiden Substraten erreicht.

Anschliessend wurde die Probe in eine Universalzugprüfmaschine (Zwick-Roell Z010) eingespannt und gespannt, so dass eine Kraft von etwa 30 N auf die Klebstelle wirkte. Die Klebstelle wurde mit UV-Licht aus einer Punktlichtquelle (Dr. Hoenle, Wellenlängenbereich λ = 320-390 nm; Intensität 900 mW cm⁻¹) bestrahlt. Dabei wurde die Klebeverbindung bereits nach etwa 30 s aufgelöst.

## Patentansprüche

1. Verwendung eines Dentalwerkstoffs, der eine thermolabile Verbindung der Formel I enthält:
[(Z¹)ₘ-Q¹-X)]ₖ-T-[Y-Q²-(Z²)ₙ]ₗ Formel I,
in der
T für eine thermolabile Einheit steht, die mindestens eine thermolabile Gruppe auf Basis von nicht-kovalenten Wechselwirkungen mit der Formel 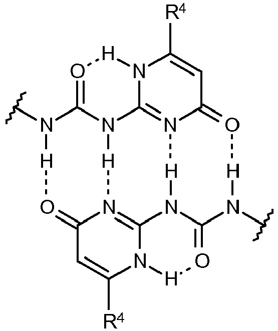 enthält,
Z¹ und Z² jeweils unabhängig für eine polymerisierbare Gruppe ausgewählt aus Vinylgruppen, CH₂=CR¹-CO-O- und CH₂=CR¹-CO-NR²- stehen,
Q¹ jeweils unabhängig entfällt oder für einen (m+1)-wertigen linearen oder verzweigten aliphatischen C₁-C₂₀-Rest steht, der durch -O-, -S-, -CO-O-, -O-CO-, -CO-NR³-, -NR³-CO-, -O-CO-NR³-, -NR³-CO-O- oder -NR³-CO-NR³-unterbrochen sein kann,
Q² jeweils unabhängig entfällt oder für einen (n+1)-wertigen linearen oder verzweigten aliphatischen C₁-C₂₀-Rest steht, der durch -O-, -S-, -CO-O-, -O-CO-, -CO-NR³-, -NR³-CO-, -O-CO-NR³-, -NR³-CO-O- oder -NR³-CO-NR³-unterbrochen sein kann,
X und Y jeweils unabhängig entfallen oder für -O-, -S-, -CO-O-, -O-CO-, -CO-NR³- oder -NR³-CO- stehen,
R, R¹, R² und R³ jeweils unabhängig für H oder einen C₁-C₇-Alkyl-Rest stehen,
R⁴ H oder ein C₁-C₁₀-Alkyl-Rest ist,
k und l jeweils unabhängig 0, 1, 2 oder 3 sind und
m und n jeweils unabhängig 1, 2 oder 3 sind,
als Adhäsiv oder Zement.

2. Verwendung nach Anspruch 1, bei der die thermolabile Einheit T eine thermolabile Gruppe ist und vorzugsweise die Formel -T¹··T²- aufweist, in der T¹ und T² für komplementäre Gruppen stehen, die durch thermolabile nicht-kovalente Wechselwirkungen miteinander verbunden sind.

3. Verwendung nach Anspruch 1, bei der die thermolabile Einheit T eine oligomere oder polymere Einheit ist, die mindestens eine thermolabile Gruppe und mindestens eine oligomere, homopolymere oder heteropolymere Kette P enthält, wobei die Kette P vorzugsweise ausgewählt ist aus der Gruppe bestehend aus Vinyl-, Dien-, Polykondensations- und Polyadditionsoligomeren und -polymeren, Copolymeren und Mischungen davon und/oder eine zahlenmittlere Molmasse von 200 bis 20000 g/mol aufweist.

4. Verwendung nach Anspruch 3, bei der die thermolabile Einheit T die Formel III oder IV hat:
[-P-(X¹-Q-Y¹-T¹··T²-Y²-Q-X²-)_{q}]ₚ Formel III,
[-P-(X¹-Q-Y¹-T¹-Y²-Q-X²-)_{q}]ₚ [(-X²-Q-Y²-T²-Y¹-Q-X¹)_{q}-P-]ₚ Formel IV,
in denen
P jeweils unabhängig für eine oligomere, homopolymere oder heteropolymere Kette steht, die vorzugsweise eine zahlenmittlere Molmasse von 200 bis 20000 g/mol aufweist,
T¹ und T² für Gruppen stehen, die durch thermolabile nicht-kovalente Wechselwirkungen miteinander verbunden sind,
Q jeweils unabhängig entfällt oder für einen zweiwertigen linearen, verzweigten und/oder cyclischen C₁-C₂₀-Rest steht, der ganz oder teilweise ungesättigt und/oder aromatisch sein kann und durch -O-, -S-, -CO-O-, -O-CO-, -CO-NR³-, -NR³-CO-, -O-CO-NR³-, -NR³-CO-O- oder -NR³-CO-NR³- unterbrochen sein kann,
X¹ und X² jeweils unabhängig entfallen oder für -O-, -S-, -CO-O-, -O-CO-, -CO-NR³-, -NR³-CO-, -O-CO-NR³-, -NR³-CO-O- oder -NR³-CO-NR³- stehen,
Y¹ und Y² jeweils unabhängig entfallen oder für -O-, -S-, -CO-O-, -O-CO-, -CO-NR³-, -NR³-CO-, -O-CO-NR³-, -NR³-CO-O- oder -NR³-CO-NR³- stehen,
p jeweils unabhängig 1 bis 100 ist und
q jeweils unabhängig 1, 2, 3 oder 4 ist.

5. Verwendung nach einem der vorhergehenden Ansprüche, bei der der Dentalwerkstoff ein oder mehrere radikalisch polymerisierbare Monomere enthält und vorzugsweise Methyl-, Ethyl-, Hydroxyethyl-, Butyl-, Benzyl-, Tetrahydrofurfuryl- oder Isobornyl(meth)acrylat, Bisphenol-A-di(meth) acrylat, Bis-GMA, UDMA, Di-, Tri- oder Tetraethylenglycoldi(meth)acrylat, Trimethylolpropantri(meth)acrylat, Pentaerythrittetra(meth)acrylat, Glycerindi(meth)acrylat, 1,4-Butandioldi(meth)acrylat, 1,10-Decandioldi(meth)acrylat, 1,12-Dodecandioldi(meth)acrylat,
und/oder
ein oder mehrere N-mono- oder -disubstitiuierte Acrylamide, N-Ethylacrylamid, N,N-Dimethacrylamid, N-(2-Hydroxyethyl)-acrylamid, N-Methyl-N-(2-hydroxyethyl)acrylamid, ein oder mehrere N-monosubstituierte Methacrylamide, N-Ethylmethacrylamid, N-(2-Hydroxyethyl)methacrylamid, N-Vinylpyrrolidon, ein oder mehrere vernetzende Allylether,
und/oder
ein oder mehrere vernetzende Pyrrolidone, 1,6-Bis(3-vinyl-2-pyrrolidonyl)-hexan, ein oder mehrere vernetzende Bisacrylamide, Methylen- oder Ethylenbisacrylamid, ein oder mehrere vernetzende Bis(meth)acrylamide, N,N'-Diethyl-1,3-bis(acrylamido)-propan, 1,3-Bis-(methacrylamido)-propan, 1,4-Bis-(acrylamido)-butan, 1,4-Bis(acryloyl)-piperazin,
und/oder
ein oder mehrere thermolabile Vernetzermonomere oder eine Mischung davon enthält.

6. Verwendung nach einem der vorhergehenden Ansprüche, bei der der Dentalwerkstoff ein oder mehrere radikalisch polymerisierbare, säuregruppenhaltige Monomere enthält und vorzugsweise Maleinsäure, Acrylsäure, Methacrylsäure, 2-(Hydroxymethyl)-acrylsäure, 4-(Meth)acryloyloxyethyltrimellitsäureanhydrid, 10-Methacryloyloxydecylmalonsäure, N-(2-Hydroxy-3-methacryloyloxypropyl)-N-phenylglycin, 4-Vinylbenzoesäure,
und/oder
Vinylphosphonsäure, 4-Vinylphenylphosphonsäure, 4-Vinylbenzylphosphonsäure, 2-Methacryloyloxyethylphosphonsäure, 2-Methacrylamidoethylphosphonsäure, 4-Methacrylamido-4-methyl-pentylphosphonsäure, 2-[4-(Dihydroxyphosphoryl)-2-oxa-butyl]-acrylsäure, 2-[4-(Dihydroxyphosphoryl)-2-oxa-butyl]-acrylsäureethyl- oder -2,4,6-trimethylphenylester,
und/oder
2-Methacryloyloxypropylmono- oder -dihydrogenphosphat, 2-Methacryloyloxyethylphenyl-hydrogenphosphat, Dipentaerythritolpentamethacryloyloxyphosphat, 10-Methacryloyloxydecyl-dihydrogenphosphat, Phosphorsäuremono-(1-acryloyl-piperidin-4-yl)-ester, 6-(Methacrylamido)hexyldihydrogenphosphat, 1,3-Bis-(N-acryloyl-N-propyl-amino)-propan-2-yldihydrogenphosphat,
und/oder
Vinylsulfonsäure, 4-Vinylphenylsulfonsäure, 3-(Methacrylamido)-propylsulfonsäure,
oder eine Mischung davon enthält.

7. Verwendung nach einem der vorhergehenden Ansprüche, bei der der Dentalwerkstoff einen Initiator für die radikalische Polymerisation enthält.

8. Verwendung nach einem der vorhergehenden Ansprüche, bei der der Dentalwerkstoff ein thermisch gasfreisetzendes Additiv enthält.

9. Verwendung nach einem der vorhergehenden Ansprüche, bei der der Dentalwerkstoff ein Additiv enthält, das eingestrahlte elektromagnetische Strahlung in Wärme umwandeln kann.

10. Verwendung nach einem der vorhergehenden Ansprüche, bei der der Dentalwerkstoff organischen und/oder anorganischen Füllstoff enthält.

11. Verwendung nach einem der vorhergehenden Ansprüche, bei der der Dentalwerkstoff
a) 0,1 bis 50 Gew.-%, insbesondere 1 bis 40 Gew.-%, bevorzugt 2 bis 30 Gew.-% und besonders bevorzugt 5 bis 30 Gew.-% Verbindung der Formel I,
b) 0,01 bis 10 Gew.-%, bevorzugt 0,1 bis 3,0 Gew.-% und besonders bevorzugt 0,2 bis 2 Gew.-% Initiator,
c) 0 bis 80 Gew.-%, bevorzugt 1 bis 60 Gew.-% und besonders bevorzugt 5 bis 50 Gew.-% Co-Monomer,
d) 0 bis 30 Gew.-%, bevorzugt 0,5 bis 15 Gew.-% und besonders bevorzugt 1 bis 5 Gew.-% Haftmonomer,
e) 0 bis 80 Gew.-% Füllstoff,
f) 0 bis 70 Gew.-% Lösungsmittel
enthält.

12. Verwendung nach Anspruch 10 oder 11 als Adhäsiv, bei der der Dentalwerkstoff 0 bis 20 Gew.-% Füllstoff enthält.

13. Verwendung nach Anspruch 10 oder 11 als Zement, bei der der Dentalwerkstoff 20 bis 80 Gew.-% Füllstoff enthält.

14. Verwendung einer Verbindung der Formel I wie in einem der Ansprüche 1 bis 4 definiert oder einer Zusammensetzung wie in einem der Ansprüche 5 bis 13 definiert zur Herstellung eines Adhäsivs oder Zements.

## Claims

1. Use of a dental material as an adhesive or cement which comprises a thermolabile compound of Formula I:
[(Z¹)ₘ-Q¹-X)ₖ-T-[Y-Q²-(Z²)ₙ]ₗ Formula I,
in which
T stands for a thermolabile entity that comprises at least one thermolabile group based on non-covalent interactions having the Formula 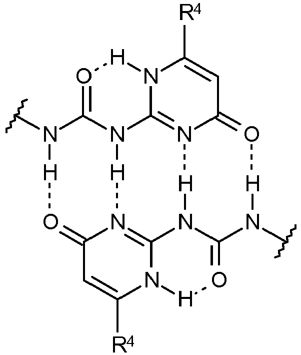
Z¹ and Z² each independently of one another stand for a polymerisable group selected from vinyl groups, CH₂=CR¹-CO-O- and CH₂=CR¹-CO-NR²-,
Q¹ each independently is missing or stands for an (m+1)-valent linear or branched aliphatic C₁-C₂₀ group that can be interrupted by -O-,-S-, -CO-O-, -O-CO-, -CO-NR³-, -NR³-CO-, -O-CO-NR³-, -NR³-CO-O- or -NR³-CO-NR³-,
Q² each independently is missing or stands for an (n+1)-valent linear or branched aliphatic C₁-C₂₀ group that can be interrupted by -O-, - S-, -CO-O-, -O-CO-, -CO-NR³-, -NR³-CO-, -O-CO-NR³-, -NR³-CO-O- or -NR³-CO-NR³-,
X and Y each independently are missing or stand for -O-, -S-, -CO-O-,-O-CO-, -CO-NR³- or -NR³-CO-,
R, R¹, R² and R³ each independently stand for H or a C₁-C₇ alkyl group,
R⁴ is H or a C₁-C₁₀ alkyl group,
k and l each independently are 0, 1, 2 or 3, and
m and n each independently are 1, 2 or 3.

2. Use according to claim 1, in which the thermolabile entity T is a thermolabile group and preferably possesses the Formula -T^{1··}T²-, in which T¹ and T² stand for complementary groups that are bonded together by thermolabile non-covalent interactions.

3. Use according to claim 1, in which the thermolabile entity T is an oligomeric or polymeric entity that comprises at least one thermolabile group and at least one oligomeric, homopolymeric or heteropolymeric chain P, wherein the chain P is preferably selected from the group consisting of vinyl-, diene-, polycondensation- and polyaddition-oligomers and -polymers, copolymers and mixtures thereof and/or exhibits a number-average molecular weight of 200 to 2000 g/mol.

4. Use according to claim 3, in which the thermolabile entity T has the Formula III or IV:
[-P-(X¹-Q-Y¹-T^{1··}T²-Y²-Q-X²-)_{q}]ₚ Formula III,
[-P-(X¹-Q-Y¹-T¹-Y²-Q-X²-)_{q}]ₚ [(-X²-Q-Y²-T²-Y¹-Q-X¹)_{q}-P-]ₚ Formula IV,
in which
P each independently stands for an oligomeric, homopolymeric or heteropolymeric chain that preferably exhibits a number-average molecular weight of 200 to 20000 g/mol,
T¹ and T² stand for groups that are bonded together by thermolabile non-covalent interactions,
Q each independently is missing or stands for a divalent linear, branched and/or cyclic C₁-C₂₀ group that can be wholly or partially unsaturated and/or aromatic and can be interrupted by -O-, -S-,-CO-O-, -O-CO-, -CO-NR³-, -NR³-CO-, -O-CO-NR³-, -NR³-CO-O- or -NR³-CO-NR³-,
X¹ and X² each independently are missing or stand for -O-, -S-, -CO-O-,-O-CO-, -CO-NR³-, -NR³-CO-, -O-CO-NR³-, -NR³-CO-O- or -NR³-CO-NR³-,
Y¹ and Y² each independently are missing or stand for -O-, -S-, -CO-O-,-O-CO-, -CO-NR³-, -NR³-CO-, -O-CO-NR³-, -NR³-CO-O- or -NR³-CO-NR³-,
p each independently is 1 to 100 and
q each independently is 1, 2, 3 or 4.

5. Use according to one of the preceding claims, in which the dental material comprises one or more radically polymerisable monomers and preferably comprises
methyl, ethyl, hydroxyethyl, butyl, benzyl, tetrahydrofurfuryl or isobornyl (meth)acrylate, bisphenol-A-di(meth)acrylate, bis-GMA, UDMA, di-, tri- or tetraethylene glycol di(meth)acrylate, trimethylolpropane tri(meth)acrylate, pentaerythritol tetra(meth)acrylate, glycerol di(meth)acrylate, 1,4-butanediol di(meth)acrylate, 1,10-decanediol di(meth)acrylate, 1,12-dodecanediol di(meth)acrylate,
and/or
one or more *N*-mono- or *N*-disubstituted acrylamides, *N*-ethylacrylamide, *N,N*-dimethacrylamide, *N*-(2-hydroxyethyl)acrylamide, *N*-methyl-*N*-(2-hydroxyethyl)acrylamide, one or more *N*-monosubstituted methacrylamides, *N*-ethylmethacrylamide, *N-*(2-hydroxyethyl)methacrylamide, *N*-vinyl pyrrolidone, one or more cross-linking allyl ethers,
and/or
one or more cross-linking pyrrolidones, 1,6-bis(3-vinyl-2-pyrrolidonyl)hexane, one or more cross-linking bisacrylamides, methylene or ethylenebisacrylamide, one or more cross-linking bis(meth)acrylamides, *N,N'*-diethyl-1,3-bis(acrylamido)propane, 1,3-bis(methacrylamido)propane, 1,4-bis(acrylamido)butane, 1,4-bis(acryloyl)piperazine,
and/or
one or more thermolabile cross-linking monomers
or a mixture thereof.

6. Use according to one of the preceding claims, in which the dental material comprises one or more radically polymerisable, acid group-containing monomers and preferably
maleic acid, acrylic acid, methacrylic acid, 2-(hydroxymethyl)acrylic acid, 4-(meth)acryloyloxyethyltrimellitic anhydride, 10-methacryloyloxydecylmalonic acid, *N*-(2-hydroxy-3-methacryloyloxypropyl)-*N*-phenylglycine, 4-vinylbenzoic acid,
and/or
vinylphosphonic acid, 4-vinylphenylphosphonic acid, 4-vinylbenzylphosphonic acid, 2-methacryloyloxyethylphosphonic acid, 2-methacrylamidoethylphosphonic acid, 4-methacrylamido-4-methylpentylphosphonic acid, 2-[4-(dihydroxyphosphoryl)-2-oxa-butyl]acrylic acid, ethyl or 2,4,6-trimethylphenyl esters of 2-[4-(dihydroxyphosphoryl)-2-oxa-butyl]acrylic acid
and/or
2-methacryloyloxypropyl mono- or dihydrogen phosphate, 2-methacryloyloxyethylphenyl hydrogen phosphate, dipentaerythritolpentamethacryloyloxy phosphate, 10-methacryloyloxydecyl dihydrogen phosphate, mono-(1-acryloyl-piperidin-4-yl) phosphate, 6-(methacrylamido)hexyl dihydrogen phosphate, 1,3-bis-(*N*-acryloyl-*N*-propylamino)propan-2-yl dihydrogen phosphate,
and/or
vinylsulfonic acid, 4-vinylphenylsulfonic acid, 3-(methacrylamido)propylsulfonic acid,
or a mixture thereof.

7. Use according to one of the preceding claims, in which the dental material comprises an initiator for radical polymerisation.

8. Use according to one of the preceding claims, in which the dental material comprises an additive that when heated releases gas.

9. Use according to one of the preceding claims, in which the dental material comprises an additive that can convert radiated electromagnetic radiation into heat.

10. Use according to one of the preceding claims, in which the dental material comprises organic and/or inorganic filler.

11. Use according to one of the preceding claims, in which the dental material comprises
a) 0.1 to 50 wt %, in particular 1 to 40 wt %, preferably 2 to 30 wt % and particularly preferably 5 to 30 wt % of the compound of Formula I,
b) 0.01 to 10 wt %, preferably 0.1 to 3.0 wt % and particularly preferably 0.2 to 2 wt % initiator,
c) 0 to 80 wt %, preferably 1 to 60 wt % and particularly preferably 5 to 50 wt % comonomer,
d) 0 to 30 wt %, preferably 0.5 to 15 wt % and particularly preferably 1 to 5 wt % adhesive monomer,
e) 0 to 80 wt % filler,
f) 0 to 70 wt % solvent.

12. Use according to claim 10 or 11 for use as an adhesive, in which the dental material comprises 0 to 20 wt % filler.

13. Use according to claim 10 or 11 as a cement, in which the dental material comprises 20 to 80 wt % filler.

14. Use of a compound of Formula I as defined in one of claims 1 to 4, or of a composition as defined in one of claims 5 to 13, for the preparation of an adhesive or cement.

## Revendications

1. Utilisation d'un matériau dentaire qui contient un composé thermolabile de formule I :
Formule I : [(Z¹)ₙ-Q¹-X)]ₖ-T-[Y-Q²-(Z²)ₙ]ₗ
dans laquelle
- T représente un fragment thermolabile qui comporte au moins un groupe thermolabile reposant sur des interactions non-covalentes, de formule 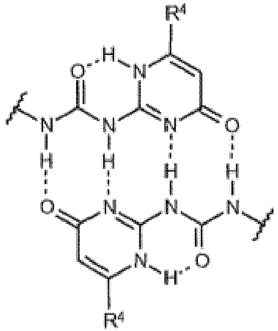
- Z¹ et Z² représentent, indépendamment en chaque occurrence, un groupe polymérisable choisi parmi le groupe vinyle et les groupes de formule CH₂=CR¹-CO-O- ou CH₂=CR¹-CO-NR²-,
- Q¹, indépendamment en chaque occurrence, ne représente rien ou représente un groupe aliphatique en C₁-C₂₀, linéaire ou ramifié, de valence m+1, au sein duquel peut être intercalé un chaînon ou un raccord symbolisé par -O-, -S-, -CO-O-, -O-CO-, -CO-NR³-, -NR³-CO-, -O-CO-NR³-, -NR³-CO-O- ou -NR³-CO-NR³-,
- Q², indépendamment en chaque occurrence, ne représente rien ou représente un groupe aliphatique en C₁-C₂₀, linéaire ou ramifié, de valence n+1, au sein duquel peut être intercalé un chaînon ou un raccord symbolisé par -O-, -S-, -CO-O-, -O-CO-, -CO-NR³-, -NR³-CO-, -O-CO-NR³-, -NR³-CO-O- ou -NR³-CO-NR³-,
- X et Y, indépendamment en chaque occurrence, ne représentent rien ou représentent un chaînon ou un raccord symbolisé par -O-, -S-, -CO-O-, -O-CO-, -CO-NR³- ou -NR³-CO-,
- R, R¹, R² et R³ représentent, indépendamment en chaque occurrence, un atome d'hydrogène ou un groupe alkyle en C₁-C₇,
- R⁴ représente un atome d'hydrogène ou un groupe alkyle en C₁-C₁₀,
- les indices k et l valent chacun, indépendamment, 0, 1, 2 ou 3,
- et les indices m et n valent chacun, indépendamment, 1, 2 ou 3,
en tant qu'adhésif ou ciment.

2. Utilisation conforme à la revendication 1, dans laquelle le fragment thermolabile symbolisé par T est un groupe thermolabile et présente de préférence la formule -T₁··T₂- dans laquelle T₁ et T₂ représentent des groupes complémentaires qui sont liés l'un à l'autre grâce à des interactions non-covalentes et thermolabiles.

3. Utilisation conforme à la revendication 1, dans laquelle le fragment thermolabile symbolisé par T est un fragment oligomère ou polymère qui comporte au moins un groupe thermolabile et au moins une chaîne oligomère, homopolymère ou hétéropolymère, symbolisée par P, laquelle chaîne P est choisie de préférence dans l'ensemble constitué par les oligomères et polymères vinyliques, de diène, de polycondensation ou de polyaddition, leurs copolymères et leurs mélanges et/ou présente une masse molaire moyenne en nombre valant de 200 à 20 000 g/mol.

4. Utilisation conforme à la revendication 3, dans laquelle le fragment thermolabile T est de formule III ou IV :
Formule III : [-P-(X¹-Q-Y¹-T¹··T²-Y²-Q-X²-)_{q}]ₚ
Formule IV : [-P-(X¹-Q-Y¹-T¹-Y²-Q-X²-)_{q}]ₚ [(-X²-Q-Y²-T²-Y¹-Q-X¹)_{q}-P-]ₚ
dans lesquelles formules
- P, indépendamment en chaque occurrence, représente une chaîne oligomère, homopolymère ou hétéropolymère, qui présente de préférence une masse molaire moyenne en nombre valant de 200 à 20 000 g/mol,
- T¹ et T² représentent des groupes qui sont liés l'un à l'autre grâce à des interactions non-covalentes et thermolabiles,
- Q, indépendamment en chaque occurrence, ne représente rien ou représente un groupe en C₁-C₂₀ divalent, linéaire, ramifié ou cyclique, qui peut être partiellement ou complètement insaturé et/ou aromatique et au sein duquel peut être intercalé un chaînon ou un raccord symbolisé par -O-, -S-, -CO-O-, -O-CO-, -CO-NR³-, -NR³-CO-, -O-CO-NR³-, -NR³-CO-O- ou -NR³-CO-NR³-,
- X¹ et X², indépendamment en chaque occurrence, ne représentent rien ou représentent un chaînon ou un raccord symbolisé par -O-, -S-, -CO-O-, -O-CO-, -CO-NR³-, -NR³-CO-, -O-CO-NR³-, -NR³-CO-O- ou -NR³-CO-NR³-,
- Y¹ et Y², indépendamment en chaque occurrence, ne représentent rien ou représentent un chaînon ou un raccord symbolisé par -O-, -S-, -CO-O-, -O-CO-, -CO-NR³-, -NR³-CO-, -O-CO-NR³-, -NR³-CO-O- ou -NR³-CO-NR³-,
- les indices p valent chacun, indépendamment, de 1 à 100,
- et les indices q valent chacun, indépendamment, 1, 2, 3 ou 4.

5. Utilisation conforme à l'une des revendications précédentes, dans laquelle le matériau dentaire contient un ou plusieurs monomère(s) polymérisable(s) par voie radicalaire, et de préférence,
- de l'acrylate ou du méthacrylate de méthyle, d'éthyle, d'hydroxy-éthyle, de butyle, de benzyle, de tétrahydrofuryle ou d'isobornyle, du di(méth)acrylate de bisphénol A, du bis-GMA, de l'UDMA, du di(méth)acrylate de diéthylèneglycol, de triéthylèneglycol ou de tétraéthylèneglycol, du tri(méth)acrylate de triméthylolpropane, du tétra(méth)acrylate de pentaérythritol, du di(méth)acrylate de glycérol, du di(méth)acrylate de butane-1,4-diol, du di(méth)acrylate de décane-1,10-diol, du di(méth)acrylate de dodécane-1,12-diol,
- et/ou un ou plusieurs acrylamide(s) portant un ou deux substituant(s) placé(s) sur l'atome d'azote, N-éthyl-acrylamide, N,N-diméthyl-acrylamide, N-(2-hydroxy-éthyl)-acrylamide, N-méthyl-N-(2-hydroxy-éthyl)-acrylamide, un ou plusieurs méthacrylamide(s) portant un substituant placé sur l'atome d'azote, N-éthyl-méthacrylamide, N-(2-hydroxy-éthyl)-méthacrylamide, de la N-vinyl-pyrrolidone, un ou plusieurs éther(s) d'allyle promoteur(s) de réticulation,
- et/ou une ou plusieurs pyrrolidone(s) promotrice(s) de réticulation, 1,6-bis(3-vinyl-2-pyrrolidonyl)-hexane, un ou plusieurs bis-acrylamide(s) promoteur(s) de réticulation, méthylène-bis-acrylamide, éthylène-bis-acrylamide, un ou plusieurs bis-(méth)acrylamide(s) promoteur(s) de réticulation, N,N'-diéthyl-1,3-bis(acrylamido)-propane, 1,3-bis(méthacrylamido)-propane, 1,4-bis(acrylamido)-butane, de la 1,4-bis(acryloyl)-pipérazine,
- et/ou un ou plusieurs monomère(s) thermolabile(s) promoteur(s) de réticulation,
ou un mélange de tels monomères.

6. Utilisation conforme à l'une des revendications précédentes, dans laquelle le matériau dentaire contient un ou plusieurs monomère(s) polymérisable(s) par voie radicalaire et comportant un groupe acide, et de préférence,
- de l'acide maléique, de l'acide acrylique, de l'acide méthacrylique, de l'acide 2-(hydroxy-méthyl)-acrylique, de l'anhydride 4-(méth)-acryloyloxy-éthyl-trimellique, de l'acide (10-méthacryloyloxy-décyl)-malonique, de la N-(2-hydroxy-3-méthacryloyloxy-propyl)-N-phényl-glycine, de l'acide 4-vinyl-benzoïque,
- et/ou de l'acide vinyl-phosphonique, de l'acide 4-vinyl-phényl-phosphonique, de l'acide 4-vinyl-benzyl-phosphonique, de l'acide 2-méthacryloyloxy-éthyl-phosphonique, de l'acide 2-méthacrylamido-éthyl-phosphonique, de l'acide 4-méthacrylamido-4-méthyl-pentyl-phosphonique, de l'acide 2-[4-(dihydroxyphosphoryl)-2-oxa-butyl]-acrylique, du 2-[4-(dihydroxyphosphoryl)-2-oxa-butyl]-acrylate d'éthyle ou de 2,4,6-triméthyl-phényle,
- et/ou du monohydrogénophosphate ou dihydrogénophosphate de 2-méthacryloyloxy-propyle, de l'hydrogénophosphate de phényle et de 2-méthacryloyloxy-éthyle, du phosphate de penta(méthacryloyloxy)-dipentaérythritol, du dihydrogénophosphate de 10-méthacryloyloxy-décyle, du phosphate de mono(1-acryloyl-pipéridin-4-yle), du dihydrogénophosphate de 6-méthacrylamido-hexyle, du dihydrogénophosphate de 1,3-bis(N-acryloyl-N-propyl-amino)-prop-2-yle,
- et/ou de l'acide vinyl-sulfonique, de l'acide 4-vinyl-benzènesulfonique, de l'acide 3-méthacrylamido-propanesulfonique,
ou un mélange de ces composés.

7. Utilisation conforme à l'une des revendications précédentes, dans laquelle le matériau dentaire contient un amorceur de polymérisation par voie radicalaire.

8. Utilisation conforme à l'une des revendications précédentes, dans laquelle le matériau dentaire contient un adjuvant qui peut libérer un gaz sous l'effet de la chaleur.

9. Utilisation conforme à l'une des revendications précédentes, dans laquelle le matériau dentaire contient un adjuvant qui peut convertir en chaleur un rayonnement électromagnétique incident.

10. Utilisation conforme à l'une des revendications précédentes, dans laquelle le matériau dentaire contient une charge organique et/ou une charge inorganique.

11. Utilisation conforme à l'une des revendications précédentes, dans laquelle le matériau dentaire contient :
a) de 0,1 à 50 % en poids, en particulier de 1 à 40 % en poids, de préférence de 2 à 30 % en poids, et mieux encore de 5 à 30 % en poids, d'un composé de formule I,
b) de 0,01 à 10 % en poids, de préférence de 0,1 à 3,0 % en poids, et mieux encore de 0,2 à 2 % en poids, d'un amorceur,
c) de 0 à 80 % en poids, de préférence de 1 à 60 % en poids, et mieux encore de 5 à 50 % en poids, d'un co-monomère,
d) de 0 à 30 % en poids, de préférence de 0,5 à 15 % en poids, et mieux encore de 1 à 5 % en poids, d'un monomère promoteur d'adhésion,
e) de 0 à 80 % en poids d'une charge,
d) et de 0 à 70 % en poids d'un solvant.

12. Utilisation conforme à la revendication 10 ou 11 en tant qu'adhésif, dans laquelle le matériau dentaire contient de 0 à 20 % en poids d'une charge.

13. Utilisation conforme à la revendication 10 ou 11 en tant que ciment, dans laquelle le matériau dentaire contient de 20 à 80 % en poids d'une charge.

14. Utilisation d'un composé de formule I, tel que défini dans l'une des revendications 1 à 4, ou d'une composition telle que définie dans l'une des revendications 5 à 13, pour la préparation d'un adhésif ou d'un ciment.
